# EUROPEAN PATENT SPECIFICATION

(11) **EP 4 093 295 B1**
(45) Date of publication and mention of the grant of the patent: **24.01.2024**
(21) Application number: 21714497.1
(22) Date of filing: 03.03.2021
(51) Int. Cl.: A61B 10/02, A61B 10/00

(54) **SLIDE-LOCK FOR BIOPSY DEVICE**
SCHIEBEVERSCHLUSS FÜR BIOPSIEVORRICHTUNG
VERROU COULISSANT POUR DISPOSITIF DE BIOPSIE

(30) Priority: 04.03.2020 US 202062985079 P
(43) Date of publication of application: 30.11.2022
(73) Proprietor: Devicor Medical Products, Inc., Cincinnati, OH 45241 (US)
(72) Inventor: GRAHAM, Mark A., Cincinnati, OH 45040 (US); HARTMAN, Douglas, Batesville, IN 47006 (US); NOCK, Andrew P., Dayton, OH 45449 (US); ROBINSON, Andrew T., Cincinnati, OH 45244 (US); BRUCE, John Kevin, Morrow, OH 4152-8265 (US)
(74) Representative: Simmons & Simmons
(86) International application number: PCT/US2021/020598
(87) International publication number: WO 2021/178489

(56) References cited:
- US-A1- 2004 167 427
- US-A1- 2008 146 965
- US-A1- 2008 200 835
- US-A1- 2010 069 787
- US-B2- 9 955 955

## Description

### BACKGROUND

Biopsy samples have been obtained in a variety of ways in various medical procedures using a variety of devices. Biopsy devices may be used under stereotactic guidance, ultrasound guidance, MRI guidance, PEM guidance, BSGI guidance, or otherwise. For instance, some biopsy devices may be fully operable by a user using a single hand, and with a single insertion, to capture one or more biopsy samples from a patient. In addition, some biopsy devices may be tethered to a vacuum module and/or control module, such as for communication of fluids (e.g., pressurized air, saline, atmospheric air, vacuum, etc.), for communication of power, and/or for communication of commands and the like. Other biopsy devices may be fully or at least partially operable without being tethered or otherwise connected with another device.

Merely exemplary biopsy devices and biopsy system components are disclosed in U.S. Pat. No. 5,526,822, entitled "Method and Apparatus for Automated Biopsy and Collection of Soft Tissue," issued June 18, 1996; U.S. Pat. No. 6,162,187, entitled "Fluid Collection Apparatus for a Surgical Device," issued December 19, 2000; U.S. Pat. No. 7,442,171, entitled "Remote Thumbwheel for a Surgical Biopsy Device," issued October 8, 2008; U.S. Pat. No. 7,938,786, entitled "Vacuum Timing Algorithm for Biopsy Device," issued May 10, 2011; U.S. Pat. No. 8,241,226, entitled "Biopsy Device with Rotatable Tissue Sample Holder," issued August 14, 2012; U.S. Pat. No. 8,764,680, entitled "Handheld Biopsy Device with Needle Firing," issued July 1, 2014; and U.S. Pat. No. 9,955,955, entitled "Biopsy Device with Slide-In Probe," issued May 1, 2018.

Additional exemplary biopsy devices and biopsy system components are disclosed in U.S. Pub. No. 2009/0131821, entitled "Graphical User Interface For Biopsy System Control Module," published May 21, 2009, now abandoned; U.S. Pub. No. 2010/0160819, entitled "Biopsy Device with Central Thumbwheel," published June 24, 2010, now abandoned; and U.S. Pub. No. 2014/0039343, entitled "Biopsy System," published February 6, 2014, now abandoned.

In some contexts, a biopsy device can be configured with a reusable holster and a disposable probe. Such a configuration can be desirable to increase economies of use by enabling more expensive parts to be reused and less expensive parts to be discarded. However, this configuration results in the need for mechanisms to couple holster to a probe. In some examples, such a mechanism can be provided by one or more hooks that engage a corresponding camming feature. Although this style of mechanism provides an advantage in terms of simplicity, it can limit feedback provided to an operator during coupling. In absence of such feedback, an operator may not be confident that the coupling between the holster and the probe is secure. Thus, there is a need to provide a mechanism for coupling a probe and a holster that provides enhanced feedback to an operator.

While several systems and methods have been made and used for obtaining a biopsy sample, it is believed that no one prior to the inventor has made or used the invention described in the appended claims.
US2008/146965 discloses a handheld biopsy device for use with a single hand, the device comprising a handpiece with a holster for detachably connecting a cutter rotation transmission and a cutter axial transmission. The preamble of claim 1 is based on this document.
US2004/167427 discloses a biopsy device with a probe for collecting tissue samples projecting from a housing with a drive for moving the tissue cutter.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a schematic view of an exemplary biopsy system including a biopsy device and a vacuum control module;
FIG. 2 depicts a perspective view of an exemplary biopsy device of the biopsy system of FIG. 1, including an exemplary probe coupled with an exemplary holster;
FIG. 3 depicts a perspective view of the biopsy device of FIG. 2, with the probe decoupled from the holster;
FIG. 4 depicts a detailed perspective view of internal components of the probe of FIG. 2;
FIG. 5 depicts a perspective cross-sectional view of the probe of FIG. 2 being attached to the holster of FIG. 2, the cross-section taken along line 5-5 of FIG. 2;
FIG. 6 depicts a perspective view of an exemplary probe for use with the holster of FIG. 2;
FIG. 7 depicts a detailed perspective view of an exemplary probe lock of the probe of FIG. 6;
FIG. 8 depicts a detailed perspective view of an exemplary lock member of the probe lock of FIG. 7;
FIG. 9 depicts a detailed perspective view of an exemplary chassis of the probe of FIG. 6;
FIG. 10 depicts a perspective view of the holster of FIG. 2, with the probe of FIG. 6 decoupled from the holster;
FIG. 11A depicts a perspective series view of the probe of FIG. 6 partially coupled to the holster of FIG. 2;
FIG. 11B depicts another perspective series view of the probe of FIG. 6 fully coupled to the holster of FIG. 2
FIG. 12A depicts a perspective series view of the probe lock of FIG. 7, the probe lock in an unlocked configuration;
FIG. 12B depicts another perspective series view of the probe lock of FIG. 7, the probe lock being actuated from the unlocked configuration by a portion of the holster of FIG. 2;
FIG. 12C depicts yet another perspective series view of the probe lock of FIG. 7, the probe lock in a locked configuration;
FIG. 13 depicts a perspective view of another exemplary probe for use with the holster of FIG. 2;
FIG. 14 depicts a detailed perspective view of an exemplary probe lock of the probe of FIG. 13; and
FIG. 15 depicts a partial cut-away perspective view of the probe of FIG. 13.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive. The scope of the invention is defined by the appended claims.

### I. Overview of Exemplary Biopsy System

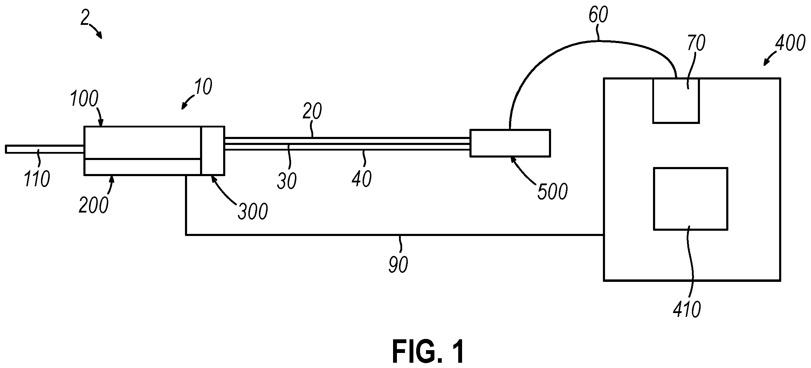

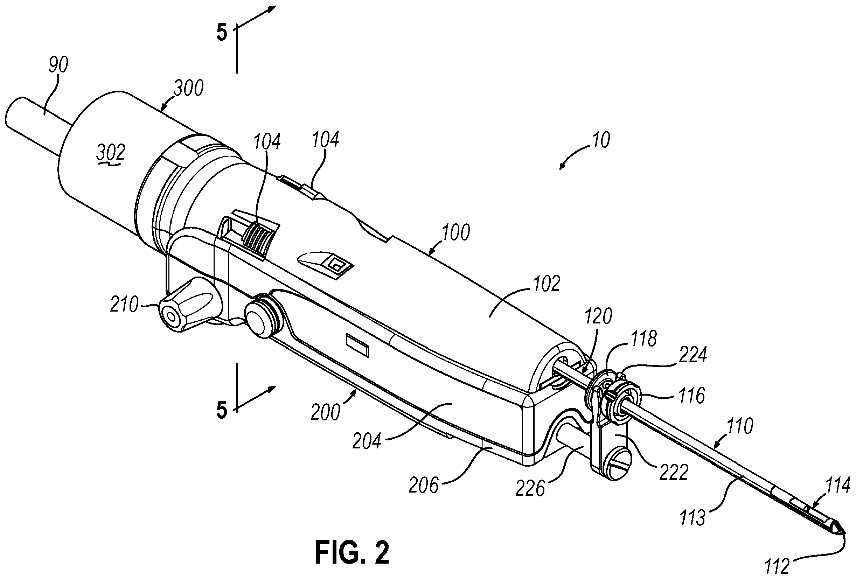

FIG. 1 depicts an exemplary biopsy system (2) comprising a biopsy device (10) and a vacuum control module (400). Biopsy device (10) of this example comprises a probe (100) and a holster (200), as shown in FIGS. 2-3. A needle (110) extends distally from probe (100), and is inserted into a patient's tissue to obtain tissue samples. These tissue samples are deposited in a tissue sample holder (300) at the proximal end of probe (100), as will also be described in greater detail below. It should also be understood that the use of the term "holster" herein should not be read as requiring any portion of probe (100) to be inserted into any portion of holster (200).

As will be discussed in greater detail below, probe (100) and holster (200) are generally configured for coupling to each other. Some variations of biopsy device (10) may include one or more sensors (not shown), in probe (100) and/or in holster (200), that is/are configured to detect when probe (100) is coupled with holster (200). Such sensors or other features may further be configured to permit only certain types of probes (100) and holsters (200) to be coupled together. In addition, or in the alternative, such sensors may be configured to disable one or more functions of probes (100) and/or holsters (200) until a suitable probe (100) and holster (200) are coupled together. In one merely illustrative example, probe (100) includes a magnet (not shown) that is detected by a hall effect sensor (not shown) or some other type of sensor in holster (200) when probe (100) is coupled with holster (200). As yet another merely illustrative example, coupling of probe (100) with holster (200) may be detected using physical contact between conductive surfaces or electrodes, using RFID technology, and/or in numerous other ways as will be apparent to those of ordinary skill in the art in view of the teachings herein. Of course, such sensors and features may be varied or omitted as desired.

Biopsy device (10) of the present example is configured to mount to a table or fixture and be used under stereotactic guidance. Of course, biopsy device (10) may instead be used under ultrasound guidance, MRI guidance, PEM guidance, BSGI guidance, or otherwise. It should also be understood that biopsy device (10) may be sized and configured such that biopsy device (10) may be operated by a single hand of a user. In particular, a user may grasp biopsy device (10), insert needle (110) into a patient's breast, and collect one or a plurality of tissue samples from within the patient's breast, all with just using a single hand. Alternatively, a user may grasp biopsy device (10) with more than one hand and/or with any desired assistance. In some settings, the user may capture a plurality of tissue samples with just a single insertion of needle (110) into the patient's breast. Such tissue samples may be pneumatically deposited in tissue sample holder (300), and later retrieved from tissue sample holder (300) for analysis. While examples described herein often refer to the acquisition of biopsy samples from a patient's breast, it should be understood that biopsy device (10) may be used in a variety of other procedures for a variety of other purposes and in a variety of other parts of a patient's anatomy (e.g., prostate, thyroid, etc.). Various exemplary components, features, configurations, and operabilities of biopsy device (10) will be described in greater detail below; while other suitable components, features, configurations, and operabilities will be apparent to those of ordinary skill in the art in view of the teachings herein.

### II. Exemplary Holster

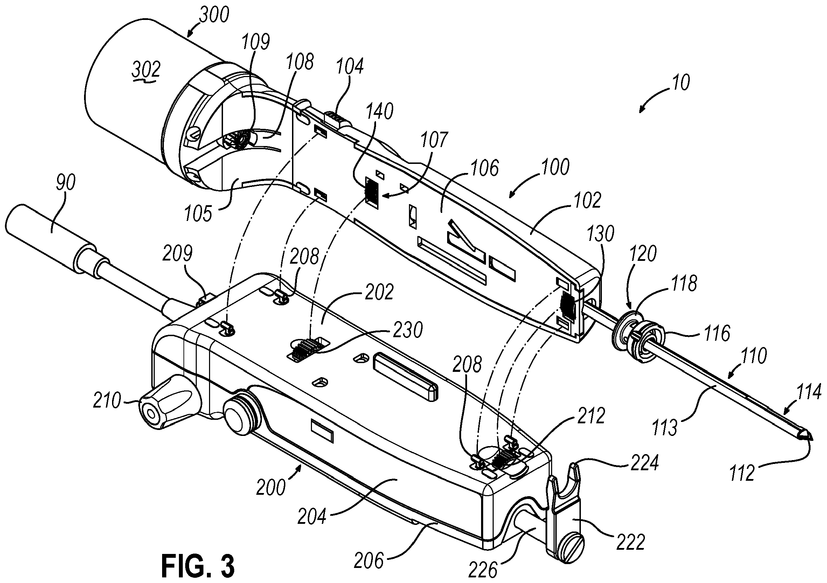

As shown in FIG. 3, holster (200) of the present example includes a top housing cover (202), side panels (204), and a housing base (206), which are fixedly secured together. Gears (212, 230) are exposed through top housing cover (202), and mesh with gears (130, 140) of probe (100) when probe (100) and holster (200) are coupled together. In particular, gears (230, 140) drive the actuation assembly of a cutter (not shown) disposed within needle (110); while gears (212, 130) are employed to rotate needle (110). A gear (not shown) is located at the proximal end of holster (200) and meshes with a corresponding gear (not shown) of probe (100) to rotate a rotatable member (not shown) of tissue sample holder (300).

As noted above, rotation of gear (212) provides rotation of needle (110) relative to probe (100). In the present example, gear (212) is rotated by rotating knob (210). In particular, knob (210) is coupled with gear (212) by a series of gears (not shown) and shafts (not shown), such that rotation of knob (210) rotates gear (212). A second knob (210) extends from the other side of holster (200). By way of example only, such a needle rotation mechanism may be constructed in accordance with the teachings of U.S. Pub. No. 2008/0214955. As another merely illustrative example, a needle rotation mechanism may be constructed in accordance with the teachings of U.S. Pub. No. 2010/0160819. In some other versions, needle (110) is rotated by a motor. In still other versions, needle (110) is simply rotated by rotating thumbwheel (116). Various other suitable ways in which rotation of needle (110) may be provided will be apparent to those of ordinary skill in the art in view of the teachings herein. It should also be understood that some versions may provide no rotation of needle (110).

Holster (200) also includes a firing rod (226) and fork (222), which couple with needle (110) and fire needle (110) distally. By way of example only, such firing may be useful in instances where biopsy device (10) is mounted to a stereotactic table fixture or other fixture, with tip (112) adjacent to a patient's breast, such that the needle firing mechanism may be activated to drive needle (110) into the patient's breast. The needle firing mechanism may be configured to drive needle (110) along any suitable range of motion, to drive tip (112) to any suitable distance relative to fixed components of probe (100).

In the present example, the needle firing mechanism is coupled with needle (110) via a firing rod (226) and a firing fork (222). Firing rod (226) and firing fork (222) are unitarily secured together. Firing fork (222) includes a pair of prongs (224) that receive hub member (120) of needle (110) therebeteween. Prongs (224) are positioned between annular flange (118) and thumbwheel (116), such that needle (110) will translate unitarily with firing rod (226) and fork (222). Prongs (224) nevertheless removably receive hub member (120), such that fork (222) may be readily secured to hub member (120) when probe (100) is coupled with holster (200); and such that hub member (120) may be readily removed from fork (222) when probe (100) is decoupled from holster (200). Prongs (224) are also configured to permit hub member (120) to rotate between prongs (224). Other suitable components, configurations, and relationships will be apparent to those of ordinary skill in the art in view of the teachings herein. The internal components of the needle firing mechanism of the present example are configured and arranged as described in U.S. Pat. No. 8,858,465, entitled "Biopsy Device with Motorized Needle Firing," issued October 14, 2014.

Holster (200) includes motors (not shown) to drive gear (230) to thereby rotate and translate cutter (150). In addition, a motor can be used to rotate a rotatable member (not shown) of tissue sample holder (300). Holster (200) also includes a motor (not shown) that is operable to drive firing rod (226), to thereby arm and fire needle (110). All motors referred to herein are contained within holster (200) in the present example and receive power from vacuum control module (400) via cable (90). In addition, data may be communicated between vacuum control module (400) and holster (200) via cable (90). As will be described in greater detail below, such data may be used by control module (400) to display certain graphical user interface screens on a touchscreen (410) integrated into control module (400). In some other versions, one or more motors are powered by one or more batteries located within holster (200) and/or probe (100). It should therefore be understood that, as with other components described herein, cable (90) is merely optional. As yet another merely illustrative variation, motors may be powered pneumatically, such that cable (90) may be substituted with a conduit communicating a pressurized fluid medium to holster (200). As still other merely illustrative variation, cable (90) may include one or more rotary drive cables that are driven by motors that are located external to holster (200). It should also be understood that two or three of the motors may be combined as a single motor. Other suitable ways in which various the motors may be driven will be apparent to those of ordinary skill in the art in view of the teachings herein.

### III. Exemplary Probe

Probe (100) of the present example includes a needle (110) extending distally from probe (100) that is inserted into a patient's tissue to obtain tissue samples. These tissue samples are deposited in a tissue sample holder (300) at the proximal end of probe (100). As shown in FIG. 1, vacuum control module (400) is coupled with probe (100) via a valve assembly (500) and tubes (20, 30, 40, 60), which is operable to selectively provide vacuum, saline, atmospheric air, and venting to probe (100). The internal components of the valve assembly of the present example are configured and arranged as described in U.S. Pub. No. 2013/0218047, entitled "Biopsy Device Valve Assembly," published August 22, 2013.

Probe (100) also includes a chassis (106) and a top housing (102), which are fixedly secured together. As best seen in FIG. 3, a gear (140) is exposed through an opening (107) in chassis (106), and is operable to drive cutter actuation mechanism in probe (100). As also seen in FIG. 3, another gear (130) is exposed through chassis (106), and is operable to rotate needle (110) as will be described in greater detail below. Gear (140) of probe (100) meshes with exposed gear (230) of holster (200) when probe (100) and holster (200) are coupled together. Similarly, gear (130) of probe (100) meshes with exposed gear (212) of holster (200) when probe (100) and holster (200) are coupled together.

Needle (110) of the present example comprises a cannula (113) having a tissue piercing tip (112), a lateral aperture (114) located proximal to tip (112), and a hub member (120). Tissue piercing tip (112) is configured to pierce and penetrate tissue, without requiring a high amount of force, and without requiring an opening to be preformed in the tissue prior to insertion of tip (112). Alternatively, tip (112) may be blunt (e.g., rounded, flat, etc.) if desired. By way of example only, tip (112) may be configured in accordance with any of the teachings in U.S. Pat. No. 8,801,742, entitled "Needle Assembly and Blade Assembly for Biopsy Device," issued August 12, 2014. As another merely illustrative example, tip (112) may be configured in accordance with at least some of the teachings in U.S. Pub. No. 2013/0150751. Other suitable configurations that may be used for tip (112) will be apparent to those of ordinary skill in the art in view of the teachings herein.

Lateral aperture (114) is sized to receive prolapsed tissue during operation of device (10). A hollow tubular cutter (not shown) having a sharp distal edge is located within needle (110). The cutter is operable to rotate and translate relative to needle (110) and past lateral aperture (114) to sever a tissue sample from tissue protruding through lateral aperture (114). For instance, the cutter may be moved from an extended position to a retracted position, thereby "opening" lateral aperture (114) to allow tissue to protrude therethrough; then from the retracted position back to the extended position to sever the protruding tissue. As will be described in greater detail below, needle (110) may be rotated to orient lateral aperture (114) at any desired angular position about the longitudinal axis of needle (110). Such rotation of needle (110) is facilitated in the present example by hub member (120), which is described in greater detail below.

Although not shown, it should be understood that needle (110) can include various internal components to subdivide the interior of needle (110) into multiple lumens. Such a multiple lumen configuration for needle (110) may be desirable in some examples to provide a lumen for the cutter and a lumen for atmospheric vent. An example of such a configuration is disclosed in U.S. Patent No. 7,918,803, entitled "Methods and Devices for Automated Biopsy and Collection of Soft Tissue," issued April 5, 2011. Of course, as with any other component described herein, any other suitable configurations may be used. A plurality of external openings (not shown) may also be formed in needle (110), and may be in fluid communication with any one or more lumens included therein. Such external openings may be configured in accordance with the teachings of U.S. Pub. No. 2007/0032742, entitled "Biopsy Device with Vacuum Assisted Bleeding Control," published February 8, 2007. Of course, as with other components described herein, such external openings in needle (110) are merely optional.

Hub member (120) of the present example is overmolded about needle (110), such that hub member (120) and needle (110) rotate and translate unitarily with each other. By way of example only, needle (110) may be formed of metal, and hub member (120) may be formed of a plastic material that is overmolded about needle (110) to unitarily secure and form hub member (120) to needle (110). Hub member (120) and needle (110) may alternatively be formed of any other suitable material(s), and may be secured together in any other suitable fashion. Hub member (120) includes an annular flange (118) and a thumbwheel (116). Gear (130) is slidably and coaxially disposed on a proximal portion of hub member (120) and is keyed to hub member (120), such that rotation of gear (130) will rotate hub member (120) and needle (110); yet hub member (120) and needle (110) may translate relative to gear (130). Gear (130) is rotatably driven by gear (212). Alternatively, needle (110) may be rotated by rotating thumbwheel (116). Various other suitable ways in which manual rotation of needle (110) may be provided will be apparent to those of ordinary skill in the art in view of the teachings herein. It should also be understood that rotation of needle (110) may be automated in various ways, including but not limited to the various forms of automatic needle rotation described in various references that are cited herein.

As noted above, the cutter is operable to simultaneously translate and rotate relative to needle (110) to sever a tissue sample from tissue protruding through lateral aperture (114). This rotation and translation is accomplished by rotation of gear (140). Although not shown, it should be understood that in some examples, gear (140) is coupled to one or more components to provide simultaneous translation and rotation of the cutter. By way of example only, the foregoing cutter actuation components can be configured in accordance with at least some of the teachings of U.S. Pub. No. 2008/0214955. As yet another merely illustrative example, the cutter may be rotated and/or translated using pneumatic motors, etc. Still other suitable ways in which the cutter may be actuated will be apparent to those of ordinary skill in the art in view of the teachings herein.

Tissue sample holder (300) of the present example provides a plurality of discrete chambers that are configured to receive tissue samples that are severed by the cutter and communicated proximally through a lumen defined by the cutter. In some examples, tissue sample holder (300) can include one or more tissue receiving trays (not shown) that are removably engaged with a rotatable member (not shown). In this configuration, the rotatable member can be configured for selective rotation within an outer cover (302) of tissue sample holder (300). As a result, the rotatable member can be selectively rotated to collect one or more tissue samples within each of the plurality of discrete chambers. It should be understood that the rotatable member and/or trays can be configured in numerous other ways. By way of example only, the rotatable member and/or the trays can be configured in accordance with at least some of the teachings of U.S. Pat. Pub. No. 2008/0214955. As another merely illustrative example, the rotatable member and/or the trays can be configured in accordance with at least some of the teachings of U.S. Pat. No. 8,702,623.

### IV. Exemplary Probe-Holster Coupling

As noted above, probe (100) is generally configured to be removably coupled to holster (200). As best seen in FIG. 3, holster (200) includes a set of prongs (208) that are received by the chassis (106) of probe (100) to releasably secure probe (100) to holster (200). In particular, probe (100) is first positioned on top of holster (200), just proximal to its final position relative to holster (200); then probe (100) is slid distally to fully engage prongs (208). Probe (100) also includes a set of resilient tabs (104) that may be pressed inwardly to disengage prongs (208), such that a user may simultaneously depress both tabs (104) then pull probe (100) rearwardly and away from holster (200) to decouple probe (100) from holster (200). Of course, a variety of other types of structures, components, features, etc. (e.g., bayonet mounts, latches, clamps, clips, snap fittings, etc.) may be used to provide removable coupling of probe (100) and holster (200). Furthermore, in some biopsy devices (10), probe (100) and holster (200) may be of unitary or integral construction, such that the two components cannot be separated. By way of example only, in versions where probe (100) and holster (200) are provided as separable components, probe (100) may be provided as a disposable component, while holster (200) may be provided as a reusable component. Still other suitable structural and functional relationships between probe (100) and holster (200) will be apparent to those of ordinary skill in the art in view of the teachings herein.

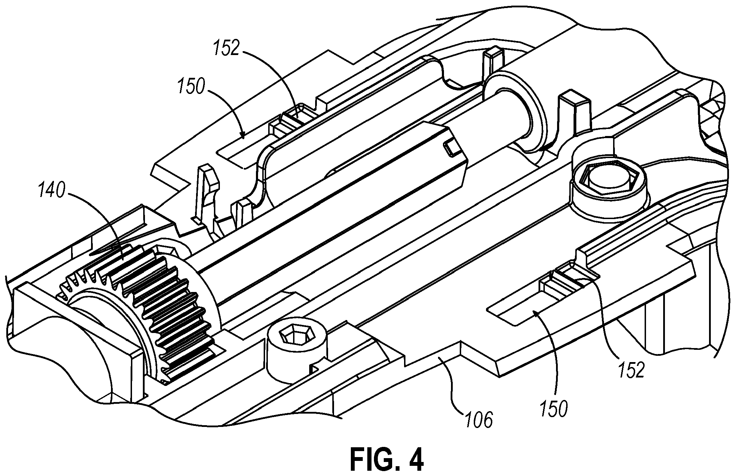

FIG. 4 shows the internal components of probe (100) used to receive prongs (208) of holster (200) in greater detail. As can be seen, chassis (106) of probe (100) defines two openings (150) positioned near the proximal end of chassis (106). Each opening (150) is sized to receive a corresponding proximally oriented prong (208) of holster (200).

Adjacent to each opening (150), chassis (106) further defines a cam feature (152) associated with each opening (150). Each cam feature (152) is generally configured to engage a corresponding prong (208) of holster (200) to provide a friction or interference fit between chassis (106) and prong (208). Thus, probe (100) is generally releasably secured to holster (200) by engagement between each cam feature (152) of probe (100) and each prong (208) of holster (200).

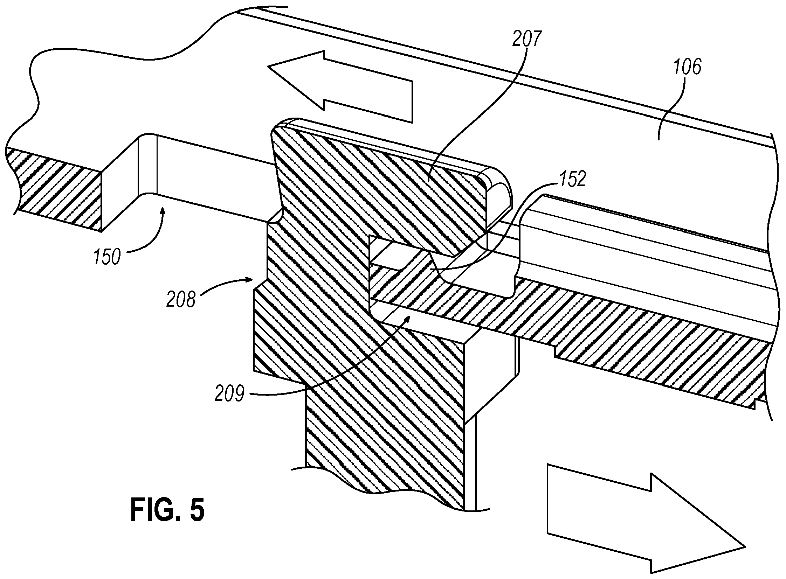

As best seen in FIG. 5, each prong (208) defines an L or hook shape that is configured to receive a portion of chassis (106) of probe (100). In particular, each prong (208) includes an overhang (207) that defines a recess (209) sized to receive chassis (106) of probe (100). During coupling between probe (100) and holster (200), probe (100) is first positioned with each prong (208) extending through each opening (150) in chassis (106). Probe (100) is then driven proximally relative to holster (200). In addition, or in the alternative, holster (200) can be driven distally relative to probe (100). Regardless, this relative movement between probe (100) and holster (200) results each cam feature (152) of chassis (106) being received within recess (209) of each prong (208) and engaging each overhang (207). Due to engagement between each cam feature (152) and each overhang (207), probe (100) is releasably coupled to holster (200) by a friction or interference fit.

### IV. Exemplary Alternative Probe with Probe Lock

As described above, probe (100) can be configured to include resilient tabs (104) to provide some locking functionality when probe (100) is coupled to holster (200). However, in some examples it may be desirable to configure a probe similar to probe (100) with locking functionality as well as mechanisms to provide an operator with affirmative feedback on such locking. Such feedback mechanisms may be desirable to increase user confidence in locking because actual coupling mechanism may be obscured by probe structures similar to top housing (102) and/or chassis (106). Although various examples of probe lock mechanisms are described below, it should be understood that various alternative probe lock mechanisms may be used as long as they fall within the scope defined by the appended claims.

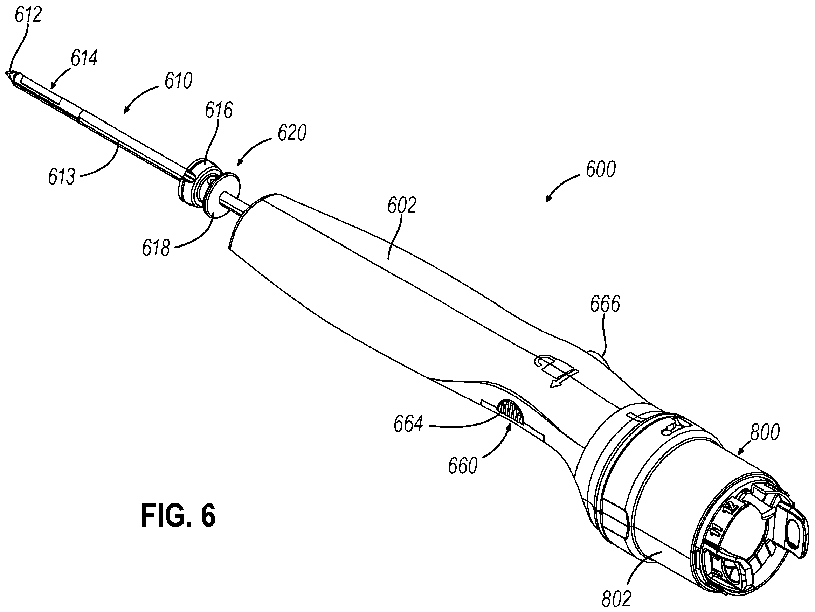

FIG. 6 shows an exemplary alternative probe (600) for use with holster (200) of biopsy system (2) described above. It should be understood that unless otherwise explicitly noted herein, probe (600) is substantially similar to probe (100) described above. For instance, as with probe (100), probe (600) of the present example includes a needle (610), a chassis (606) and a top housing (602). Needle (610) extends distally from chassis (606) and top housing (602). As with needle (110) described above, needle (610) of the present example includes a cannula (613) that defines a lateral aperture (614) and terminates at a sharp distal tip (612).

As with needle (110) described above, it should be understood that needle (610) can likewise include various internal components to subdivide the interior of needle (610) into multiple lumens. Such internal components can be configured in accordance with one or more of the teachings of U.S. Patent No. 7,918,803, entitled "Methods and Devices for Automated Biopsy and Collection of Soft Tissue," issued April 5, 2011.

As also with needle (110) described above, needle (610) of the present example can include a hub member (620) for receiving fork (222) of holster (200). Hub member (620) of the present example is substantially similar to hub member (120) described above. For instance, hub member (620) of the present example is overmolded about needle (610), such that hub member (620) and needle (610) rotate and translate unitarily with each other. Likewise, hub member (620) includes an annular flange (618) and a thumbwheel (616).

Also like with needle (110) described above, needle (610) of the present example can include a cutter (not shown) configured to rotate and translate within the interior of needle (610) to sever a tissue sample from tissue protruding through lateral aperture (614). This rotation and translation is accomplished by rotation of a gear (640). Although not shown, it should be understood that in some examples, gear (640) is coupled to one or more components to provide simultaneous translation and rotation of the cutter. By way of example only, the foregoing cutter actuation components can be configured in accordance with at least some of the teachings of U.S. Pub. No. 2008/0214955. As yet another merely illustrative example, the cutter may be rotated and/or translated using pneumatic motors, etc. Still other suitable ways in which the cutter may be actuated will be apparent to those of ordinary skill in the art in view of the teachings herein.

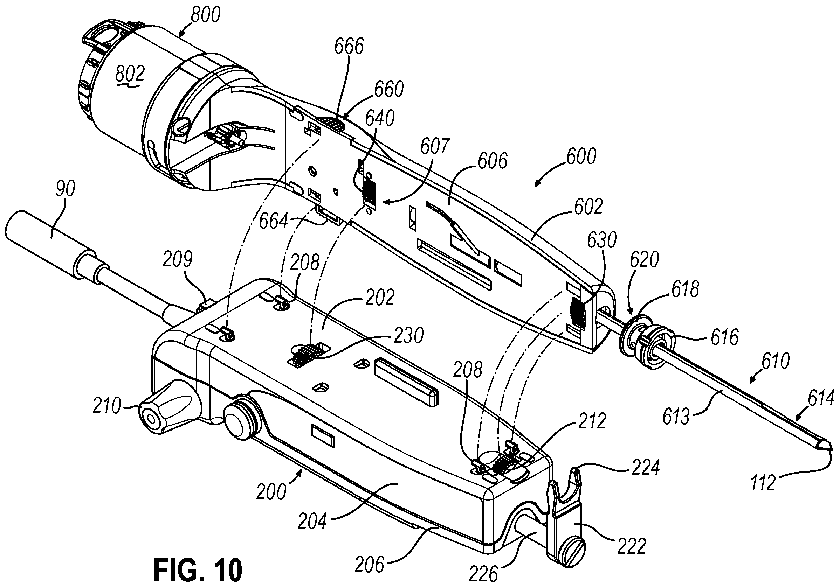

As best seen in FIG. 10, probe further includes gears (640, 630), similar to gears (140, 130) described above. In particular, gear (640) is exposed through an opening (607) in chassis (606) and is operable to drive a cutter actuation mechanism included within probe (600). As also seen in FIG. 10, gear (630) is exposed through chassis (606), and is operable to rotate needle (610). Gear (640) of probe (600) meshes with exposed gear (230) of holster (200) when probe (600) and holster (200) are coupled together. Similarly, gear (630) of probe (600) meshes with exposed gear (212) of holster (200) when probe (600) and holster (200) are coupled together.

Returning to FIG. 6, probe (600) further includes a tissue sample holder (800) similar to tissue sample holder (300) described above. As with tissue sample holder (300) described above, tissue sample holder (800) of the present example provides a plurality of discrete chambers that are configured to receive tissue samples that are severed by the cutter and communicated proximally through a lumen defined by the cutter. In some examples, tissue sample holder (800) can include one or more tissue receiving trays (not shown) that are removably engaged with a rotatable member (not shown). In this configuration, the rotatable member can be configured for selective rotation within an outer cover (802) of tissue sample holder (800). As a result, the rotatable member can be selectively rotated to collect one or more tissue samples within each of the plurality of discrete chambers. It should be understood that the rotatable member and/or trays can be configured in numerous other ways. By way of example only, the rotatable member and/or the trays can be configured in accordance with at least some of the teachings of U.S. Pat. Pub. No. 2008/0214955. As another merely illustrative example, the rotatable member and/or the trays can be configured in accordance with at least some of the teachings of U.S. Pat. No. 8,702,623.

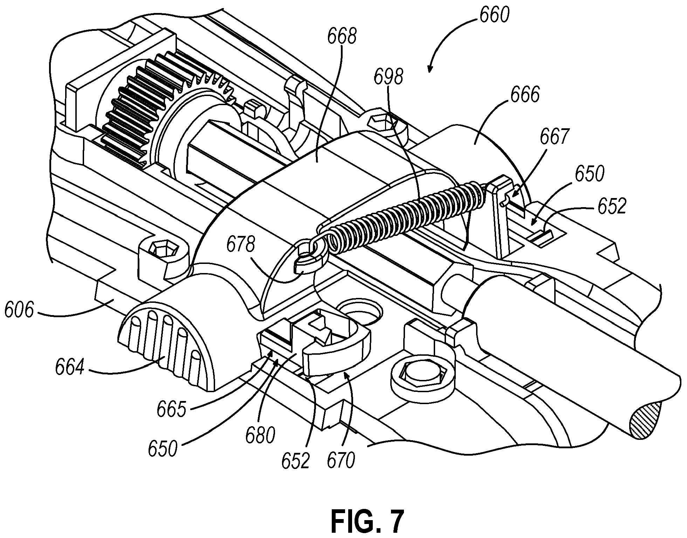

Unlike probe (100) described above, probe (600) of the present example includes a probe lock (660) that is generally configured to provide affirmative feedback to an operator when coupling probe (600) to holster (200) and also provide a locking mechanism to prevent decoupling of probe (600) from holster (200). As best seen in FIG. 7, probe lock (660) includes a lock member (662), a retainer (680), and a resilient member (698). Lock member (662) disposed within the interior of probe (600) and on the upper surface of chassis (606). As will be described in greater detail below, lock member (662) is generally configured to automatically lock when probe (600) is coupled to holster (200), while providing affirmative feedback to an operator of such locking.

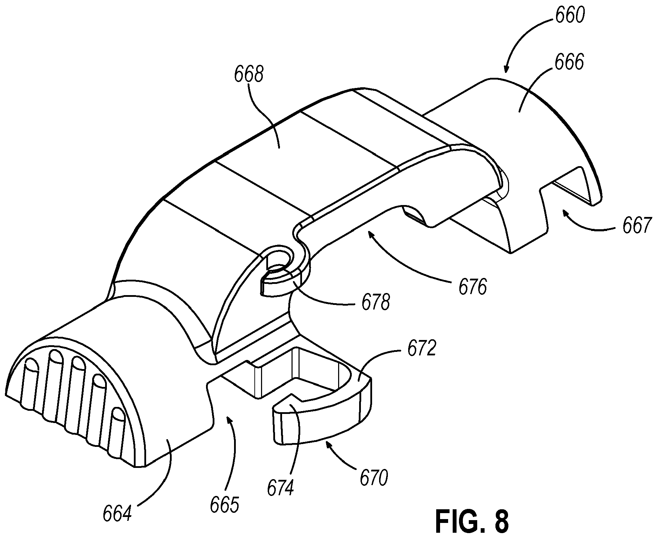

As best seen in FIG. 8, lock member (662) includes an unlock indicator (664), a lock indicator (666), and a body (668) disposed between each indicator (664, 666). As will be described in greater detail below, lock member (662) is generally configured to respond to a prong (208) of holster (200) to automatically transition from an unlocked position to a locked position while also affirmatively indicating the transition to an operator.

Each indicator (664, 666) is configured to provide an affirmative indicator to an operator related to the position of lock member (662) within probe (600) and thereby indicate the operational status probe lock (660) (e.g., locked or unlocked). For instance, in the present example, each indicator (664, 666) protrudes from an opposite side of body (668). As will be described in greater detail below, this positioning permits at least a portion of a particular indicator (664, 666) to protrude from top housing (602), while the other indicator (666, 664) is flush or retracted within top housing (602). In addition, each indicator is configured as button or actuator. As will be described in greater detail below, in some circumstances, this configuration permits either indicator (664, 666) to be used to move or otherwise actuate lock member (662) within probe (600).

Each indicator (664, 666) defines a corresponding prong gap (665, 667). As will be described in greater detail below, each prong gap (665, 667) is configured to permit a prong (208) of holster (200) to pass though chassis (606) and into the interior of probe (600) during coupling of probe (600) to holster (200). In other words, each prong gap (665, 667) is configured to provide clearance for entry of a given prong (208) of holster (200) into probe (600).

Body (668) is shaped to define a bridge gap (676) and an attachment point (678). Bridge gap (676) is sized and shaped to provide clearance for various internal components of probe (600), while still permitting lock member (662) to move within the interior of probe (600). As will be described in greater detail below, attachment point (678) is configured to provide a point attachment for resilient member (698), such that resilient member (698) can couple to body (668). As will also be described in greater detail below, attachment point (678) is generally oriented towards one side of body (668) to promote specific movement of body (668) under the influence of resilient member (698).

Body (668) further includes a flex latch (670) protruding proximally from a proximal surface of body (668). Flex latch (670) is generally configured to provide selective engagement with at least a portion of chassis (606) to thereby provide a release mechanism for lock member (662). As will be described in greater detail below, flex latch (670) is configured to be responsive to a specific prong (208) of holster (200) to cause automatic transitioning of probe lock (660) from an unlocked configuration to a locked configuration. As such, it should be understood that at least a portion of flex latch (670) can be configured to have some generally flexible yet resilient material properties.

Flex lock (670) includes a curved portion (672) and a toothed end (674). Curved portion (672) extends proximally from body (668) and then curves laterally away from the initial axis of extension. In other words, curved portion (672) generally forms a hook or catch shape. The thickness of curved portion (672) can also be configured to provide some flexibility to flex lock (670), while also providing some resiliency. As will be described in greater detail below, this configuration generally promotes the ability of flex lock (670) to flex or move in response to engagement with a specific prong (208) of holster (200).

Curved portion (672) terminates in a toothed end (674). Toothed end (674) generally provides a flat surface configured to releasably engage with at least a portion of chassis (606). As will be described in greater detail below, toothed end (674) is generally configured to engage retainer (680) to hold lock member (662) in a given position until acted upon by a specific prong (208) of holster (200).

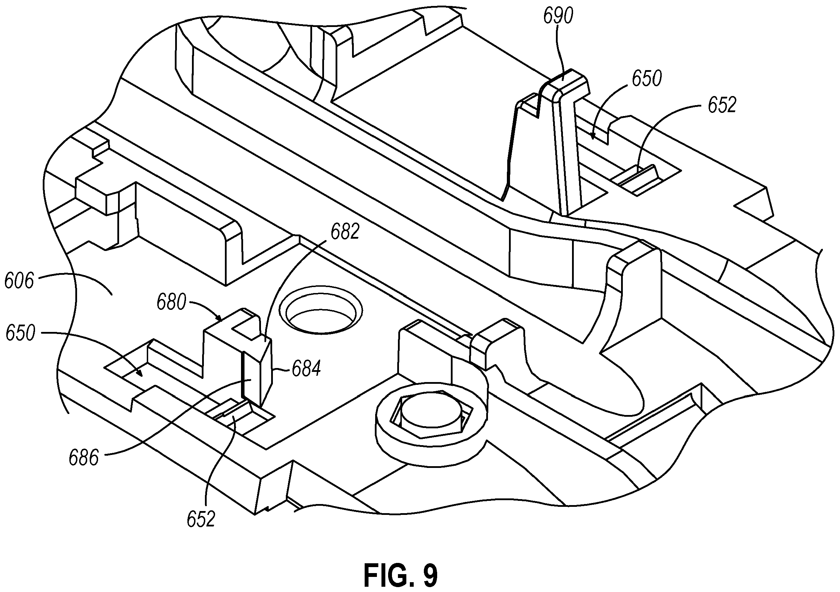

As best seen in FIG. 9, chassis (606) is generally substantially similar to chassis (106) described above. For instance, like with chassis (106), chassis (606) of the present example includes openings (650) configured to receive a particular prong (208) of holster (200). Similarly, chassis (606) also incudes a cam feature (652) adjacent to each opening (650) to provide a friction or interference fit with a selected prong (208) of holster (200).

Unlike chassis (106) described above, chassis (606) of the present example defines retainer (680) extending upwardly from a lower surface of chassis (606). Retainer (680) is positioned adjacent to an opening (650). Although retainer (680) of the present example is configured as being generally integral with chassis (606), it should be understood that in other examples retainer (680) can be a separate component fastened or otherwise secured to chassis (606).

Retainer (680) generally includes a wedge portion (682) defining a ramp (684) and a flat surface (686). As will be understood, wedge portion (682) is generally configured to engage with flex lock (670) to manipulate flex lock (670) through various positions. Thus, it should be understood that the particular geometry of wedge portion (682) is generally configured to manipulate flex lock (670). In connection with this, ramp (684) is generally configured to push flex lock (670) proximally in response to lateral movement of lock member (662) to direct flex lock (670) into engagement with flat surface (686). Thus, ramp (684) is sloped proximally towards flat surface (686). As will be described in greater detail below, flat surface (686) is configured to provide a surface for toothed end (674) of flex lock (670) to engage retainer (680).

Flex lock (670) and retainer (680) of the present example are shown as being oriented on a particular side of chassis (606). Thus, as will be described in greater detail below, both flex lock (670) and retainer (680) are configured to engage with a particular prong (208) of holster (200). However, it should be understood that in other examples, both flex lock (670) and retainer (680) can be positioned on an opposite side of chassis (606) to engage with another prong (208). Similarly, in other examples, flex lock (670) can retainer (680) can be replicated in multiple positions relative to chassis (606) to engage multiple prongs (208) of holster (200). Still, various alternative configurations for flex lock (670) and retainer (680) will be apparent to those of ordinary skill in the art in view of the teachings herein.

Chassis (606) further includes an attachment post (690). Attachment post (690) is generally configured to provide another point of attachment for resilient member (698), which extends between attachment post (690) and attachment point (678) of body (668). Thus, it should be understood that resilient member (698) is generally configured to provide tension between attachment post (690) and attachment point (678). To provide a suitable amount of tension, attachment post (690) is positioned on an opposite side of chassis (606) relative to attachment point (678).

Resilient member (698) of the present example is configured as a coil spring with hooks on each end that are configured to couple resilient member (698) to attachment point (678) and attachment post (690). Although resilient member (698) is shown as a coil spring, it should be understood that other examples can include other components configured to provide tension such as an elastomeric band or shape memory alloy. As will be described in greater detail below, resilient member (698) is generally configured to pull lock member (662) transversely across chassis (606) towards attachment post (690) to thereby transition probe lock (660) from the unlocked configuration to the locked configuration.

FIGS. 10 through 12C show an exemplary operation to couple probe (600) to holster (200). As can be seen in FIG. 10, the operation begins with probe (600) detached from holster (200). In some operations, probe (600) is provided to an operator with probe lock (660) in the unlocked configuration. In this configuration, lock member (662) is oriented such that at least a portion of unlock indicator (664) is protruding from top housing (602) of probe (600).

In other operations, probe (600) can be provided to an operator with probe lock (660) in the locked configuration. This may be an undesirable condition prior to coupling of probe (600) to holster (200) because probe lock (660) may interfere with prongs (208) of holster (200). Thus, in some operations, an operator may desire to transition probe lock (660) to the unlocked configuration prior to coupling of probe (600) to holster (200). To do so, an operator can push lock indicator (666) transversely relative to the longitudinal axis of probe (600) until unlock indicator (664) protrudes from top housing (602). At this stage, probe (600) is ready for coupling to holster (200).

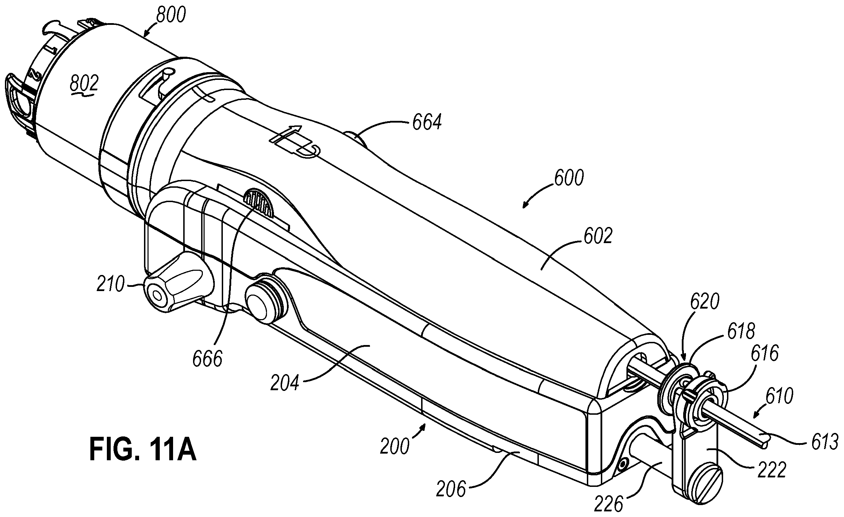

To initiate coupling of probe (600) to holster (200), an operator can align probe (600) with holster (200) such that each opening (650) of probe (600) is aligned with each prong (208) of holster (200) as shown in FIG. 10. Probe (600) is then placed into the top of holster (200) with each prong (208) of holster (200) being inserted into each opening (650) of probe (600) as can be seen by comparing FIGS. 10 and 11A.

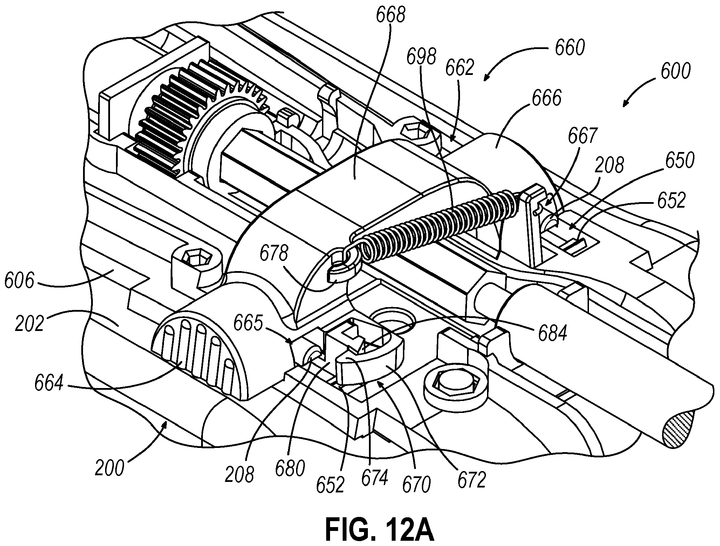

Once probe (600) is positioned as shown in FIG. 11A, probe (600) is mated with holster (200), but not yet locked in position. In particular, as can be seen in FIG. 11A and 12A, prongs (208) are merely inserted into openings (650), but not locked onto cam features (652).

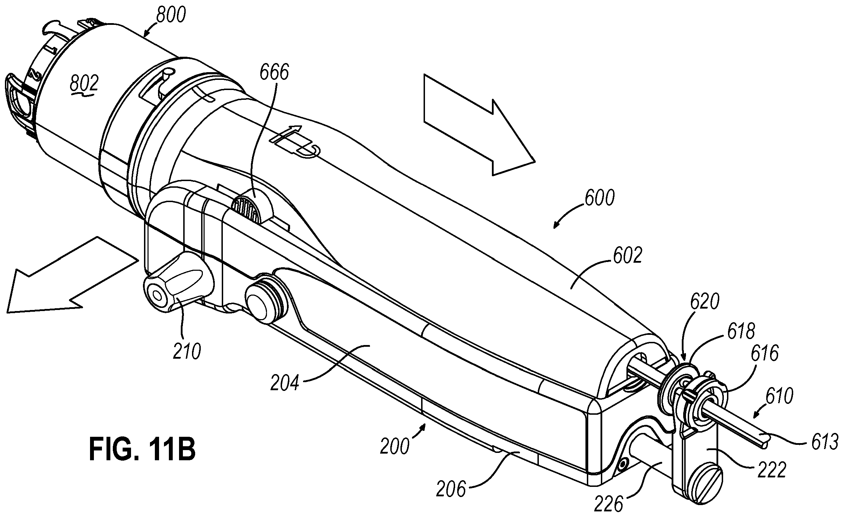

To lock each prong (208) onto each corresponding cam feature (652), probe (600) is translated distally relative to holster (200) as shown in FIG. 11B. Alternatively, holster (200) can be translated proximally relative to probe (600) or some combination of both probe (600) and holster (200) being translated relative to each other. Regardless, this proximal translation causes each prong (208) to engage each cam feature (652) to lock probe (600) to holster (200) by interference or friction fit.

During coupling of probe (600) to holster (200), probe lock (660) also operates automatically to shift from the unlocked configuration to the locked configuration. When shifting to the unlocked or locked status is indicated by protrusion of either unlock indicator (664) or lock indicator (666) from top housing (602). Additionally, in some examples, the transition from an unlocked to locked configuration can be accompanied with an audible "click," "ping," or "ding" to further provide affirmative feedback of locking.

As best seen in FIG. 12A, probe lock (660) is initially in the unlocked configuration. In this configuration, lock member (662) is oriented laterally to one side of chassis (606) such that unlock indicator (664) protrudes from the side of chassis (606). With lock member (662) in this position, flex lock (670) is engaged with retainer (680) in particular, toothed end (674) of flex lock (670) is engaged with flat surface (686) of retainer (680) to hold lock member (662) in the unlocked position. In addition, lock member (662) is held in position against the resilient bias of resilient member (698) such that resilient member (698) is in a stretched configuration.

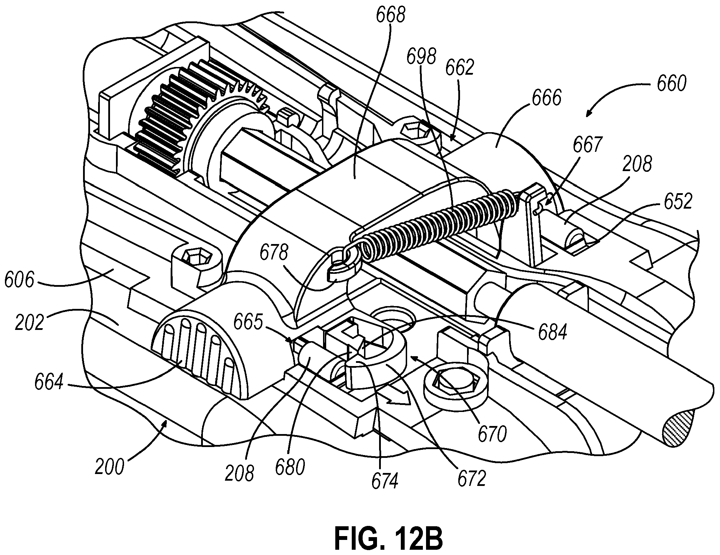

As shown in FIG. 12B, distal advancement of probe (600) causes prong (208) of holster (200) to engage toothed end (674) of flex lock (670). This engagement pushes flex lock (670) to cause bending at curved portion (672). Bending of flex lock (670) continues until toothed end (674) disengages from flat surface (686) of retainer (680).

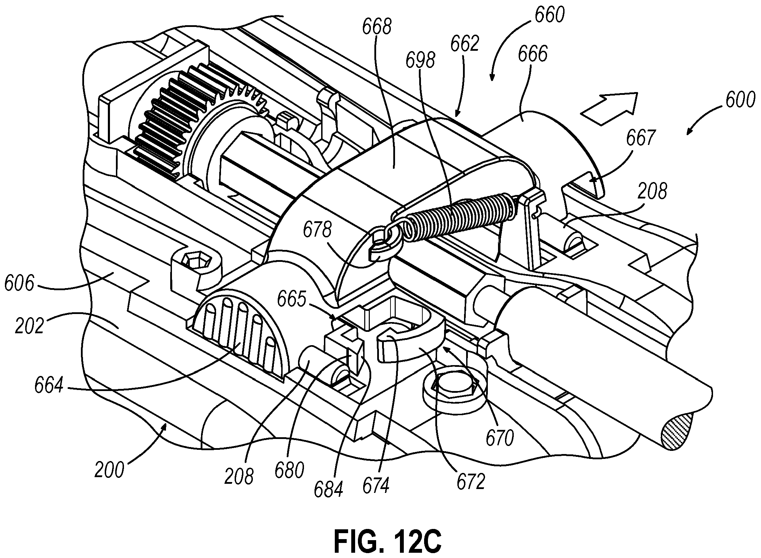

Once toothed end (674) of flex lock (670) is disengaged from flat surface (686) of retainer (680), lock member (662) is free to translate laterally within probe (600) relative to the longitudinal axis of probe (600) (e.g., perpendicular relative to the longitudinal axis). With this expanded degree of freedom, resilient member (698) can pull lock member (662) from the position shown in FIG. 12B to the position shown in FIG. 12C, which corresponds to the locked configuration.

In the locked configuration, lock member (662) is positioned such that unlock indicator (664) is positioned generally within the perimeter of chassis (606), while lock indicator (666) is positioned generally outside the perimeter of chassis (606). In this position, each prong gap (665, 667) is also positioned away from prongs (208). Consequently, each indicator (664, 666) is positioned adjacent to the distal surface of each prong (208). Thus, each indicator (664, 666) physically locks each prong (208) into engagement with cam feature (652) to prevent decoupling of probe (600) from holster (200).

Once probe (600) is coupled to holster (200) as described above, a biopsy procedure can be performed. At the conclusion of such a procedure, an operator may desire to decouple probe (600) from holster (200) for disposal of probe (600) and reuse of holster (200). To decouple probe (600) from holster (200), the procedure described above can be repeated. In particular, an operator can press lock indicator (666) to move lock member (662) back to the unlocked position shown in FIG. 12A.

Once lock member (662) is moved, unlock indicator (664) indicates to an operator that probe (600) is unlocked by generally protruding beyond the outer perimeter of chassis (606). An operator can then remove probe (600) by pulling probe (600) proximally relative to holster (200) to disengage each prong (208) from each corresponding cam feature (652).

### V. Exemplary Alternative Probe with Manual Probe Lock

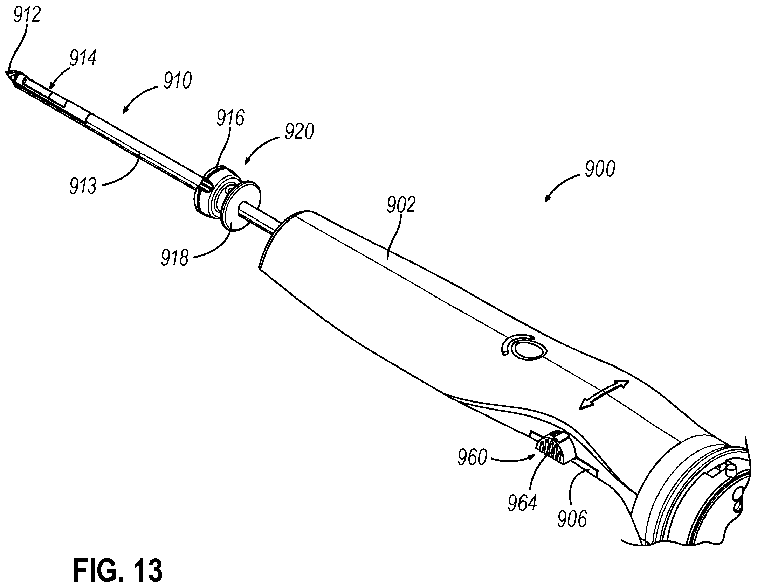

FIGS. 13-14 show another exemplary alternative probe (900) that is substantially similar to probe (600) described above. For instance, as with probe (600), probe (900) of the present example includes a needle (910), a chassis (906) and a top housing (902). Needle (910) extends distally from chassis (906) and top housing (902). As with needle (610) described above, needle (910) of the present example includes a cannula (913) that defines a lateral aperture (914) and terminates at a sharp distal tip (912).

As with needle (610) described above, it should be understood that needle (910) can likewise include various internal components to subdivide the interior of needle (910) into multiple lumens. Such internal components can be configured in accordance with one or more of the teachings of U.S. Patent No. 7,918,803, entitled "Methods and Devices for Automated Biopsy and Collection of Soft Tissue," issued April 5, 2011.

As also with needle (610) described above, needle (910) of the present example can include a hub member (920) for receiving fork (222) of holster (200). Hub member (920) of the present example is substantially similar to hub member (620) described above. For instance, hub member (920) of the present example is overmolded about needle (910), such that hub member (920) and needle (910) rotate and translate unitarily with each other. Likewise, hub member (920) includes an annular flange (918) and a thumbwheel (916).

Also like with needle (610) described above, needle (910) of the present example can include a cutter (not shown) configured to rotate and translate within the interior of needle (910) to sever a tissue sample from tissue protruding through lateral aperture (914). This rotation and translation is accomplished by rotation of a gear (940). Although not shown, it should be understood that in some examples, gear (940) is coupled to one or more components to provide simultaneous translation and rotation of the cutter. By way of example only, the foregoing cutter actuation components can be configured in accordance with at least some of the teachings of U.S. Pub. No. 2008/0214955. As yet another merely illustrative example, the cutter may be rotated and/or translated using pneumatic motors, etc. Still other suitable ways in which the cutter may be actuated will be apparent to those of ordinary skill in the art in view of the teachings herein.

Probe further includes gears (not shown) similar to gears (640, 630) described above. As with gears (640, 630), the gears in the present example can be exposed through an opening in chassis (906) to drive a cutter actuation mechanism and rotation of needle (910). As similarly discussed above with respect to gears (640, 630), each gear in the present example can mesh with a corresponding gear (212, 230) of holster (200) when probe (900) and holster (200) are coupled together.

Although not shown, it should be understood that probe (900) of the present example can be equipped with a tissue sample holder similar to tissue sample holders (300, 800) described above. As with tissue sample holders (300, 800) described above, the tissue sample holder of the present example can provide a plurality of discrete chambers that can be configured to receive tissue samples severed by the cutter and communicated proximally through a lumen defined by the cutter. In some examples, the tissue sample holder can include one or more tissue receiving trays (not shown) that are removably engaged with a rotatable member (not shown). In this configuration, the rotatable member can be configured for selective rotation within an outer cover (not shown) of the tissue sample holder. As a result, the rotatable member can be selectively rotated to collect one or more tissue samples within each of the plurality of discrete chambers. It should be understood that the rotatable member and/or trays can be configured in numerous other ways. By way of example only, the rotatable member and/or the trays can be configured in accordance with at least some of the teachings of U.S. Pat. Pub. No. 2008/0214955. As another merely illustrative example, the rotatable member and/or the trays can be configured in accordance with at least some of the teachings of U.S. Pat. No. 8,702,623.

Like probe (600) described above, probe (900) of the present example also includes a probe lock (960) that is generally configured to provide affirmative feedback to an operator when coupling probe (900) to holster (200) and also provide a locking mechanism to prevent decoupling of probe (900) from holster (200). Probe lock (960) is generally similar to probe lock (660) described above. However, unlike probe lock (660), probe lock (960) of the present example is generally configured for manual actuation rather than incorporating one or more automatic actuation features.

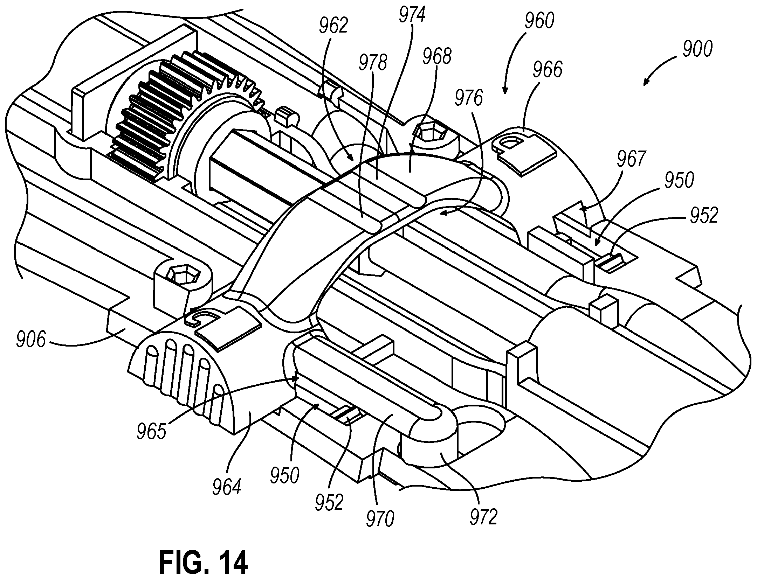

As best seen in FIG. 14, probe lock (960) includes a lock member (962), but omits structures similar to a retainer (680), and a resilient member (698). Lock member (962) disposed within the interior of probe (900) and on the upper surface of chassis (906). As will be described in greater detail below, lock member (962) is generally configured to be actuated by a user to lock when probe (900) is coupled to holster (200), while also providing affirmative feedback to an operator of such locking.

Lock member (962) includes an unlock indicator (964), a lock indicator (966), and a body (968) disposed between each indicator (964, 966). As will be described in greater detail below, lock member (962) is generally configured for manual translation within probe (900) to move from an unlocked position to a locked position while also affirmatively indicating the transition to an operator.

Each indicator (964, 966) is configured to provide an affirmative indicator to an operator related to the position of lock member (962) within probe (900) and thereby indicate the operational status probe lock (960) (e.g., locked or unlocked). For instance, in the present example, each indicator (964, 966) protrudes from an opposite side of body (968). As will be described in greater detail below, this positioning permits at least a portion of a particular indicator (964, 966) to protrude from top housing (902), while the other indicator (966, 964) is flush or retracted within top housing (902). In addition, each indicator is configured as button or actuator. As will be described in greater detail below, in some circumstances, this configuration permits either indicator (964, 966) to be used to move or otherwise actuate lock member (962) within probe (900).

Each indicator (964, 966) defines a corresponding prong gap (965, 967). As will be described in greater detail below, each prong gap (965, 967) is configured to permit a prong (208) of holster (200) to pass though chassis (906) and into the interior of probe (900) during coupling of probe (900) to holster (200). In other words, each prong gap (965, 967) is configured to provide clearance for entry of a given prong (208) of holster (200) into probe (900).

Body (968) is shaped to define a bridge gap (976), but structures similar to attachment point (678) are omitted. Bridge gap (976) is sized and shaped to provide clearance for various internal components of probe (900), while still permitting lock member (962) to move within the interior of probe (900).

Unlike body (668) described above, body (968) of the present example omits structures similar to flex latch (670). Instead, body (968) further includes a tracer arm (970) extending proximally from a proximal surface of body (968). Tracer arm (970) is generally configured to move a magnet or other component as lock member (962) moves to provide certain signals to holster (200). In particular, tracer arm (970) extends proximally from body (968) to avoid impediment of operation of any components such as prongs (208). An inverted cup (972) is positioned on the proximal end of tracer arm (970). Although not shown, it should be understood that in the present example a magnet can be disposed in cup (972). In this configuration, the magnet is slid by tracer arm (970) along the surface of chassis (906). As will be described in greater detail below, some versions of holster (200) can include a hall effect sensor or other device positioned to correspond to the magnet. Such a sensor can then send signals to vacuum control module (400) to identify the position of lock member (962) and thus the state of probe lock (960).

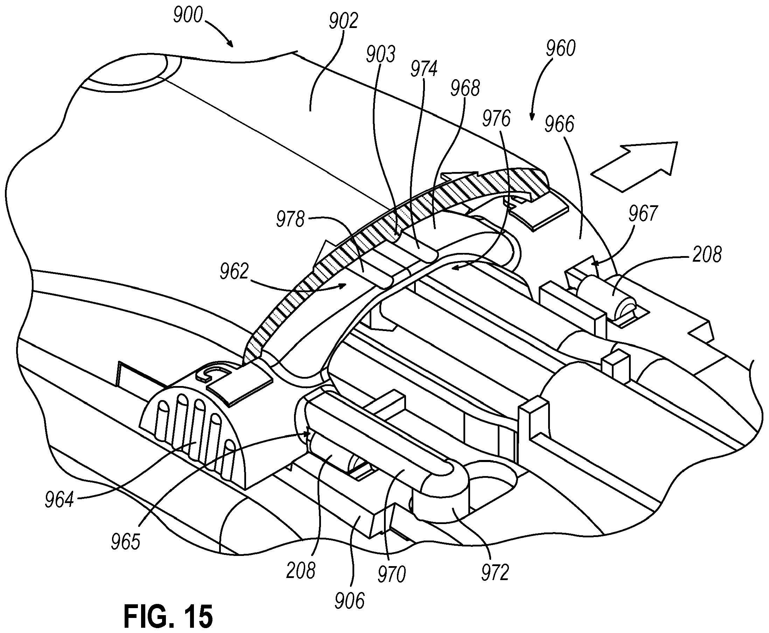

Body (968) further includes a unlock detent (974) and a lock detent (978). As best seen in FIG. 15, both unlock detent (974) and lock detent (978) are configured to engage a corresponding detent feature (903) integrated into top housing (902). As will be described in greater detail below, each detent (974, 978) is configured to releasably hold lock member (962) in a predetermined position until lock member (962) is manually actuated by an operator. In the present example, detents (974, 978) are configured as two elongate indentations on the surface of body (968), while detent feature (903) is configured as a protrusion. However, it should be understood that in other examples, this configuration can be reversed. In still other examples, detents (974, 978) and/or detent feature (903) can be configured as any other suitable resilient locking mechanism as will be apparent to those of ordinary skill in the art.

In use, probe lock (960) is used similarly to probe lock (660) described above. For instance, as similarly described above, the operation begins with probe (900) detached from holster (200). In some operations, probe (900) is provided to an operator with probe lock (960) in the unlocked configuration. In this configuration, lock member (962) is oriented such that at least a portion of unlock indicator (964) is protruding from top housing (902) of probe (900).

In other operations, probe (900) can be provided to an operator with probe lock (960) in the locked configuration. This may be an undesirable condition prior to coupling of probe (900) to holster (200) because probe lock (960) may interfere with prongs (208) of holster (200). Thus, in some operations, an operator may desire to transition probe lock (960) to the unlocked configuration prior to coupling of probe (900) to holster (200). To do so, an operator can push lock indicator (966) transversely relative to the longitudinal axis of probe (900) until unlock indicator (964) protrudes from top housing (902). At this stage, probe (900) is ready for coupling to holster (200).

To initiate coupling of probe (900) to holster (200), an operator can align probe (900) with holster (200) such that a plurality of openings (950) of probe (900) are correspondingly aligned with prongs (208) of holster (200). Probe (900) is then placed into the top of holster (200) with each prong (208) of holster (200) being inserted into each opening (950) of probe (900).

Once probe (900) is positioned with prongs (208) inside openings (950), probe (900) is mated with holster (200), but not yet locked in position. In particular, prongs (208) are merely inserted into openings (950), but not locked onto a corresponding cam feature (952) of a plurality of cam features (952).

To lock each prong (208) onto each corresponding cam feature (952), probe (900) is translated distally relative to holster (200). Alternatively, holster (200) can be translated proximally relative to probe (900) or some combination of both probe (900) and holster (200) being translated relative to each other. Regardless, this proximal translation causes each prong (208) to engage each cam feature (952) to lock probe (900) to holster (200) by interference or friction fit.

Unlike the operation described above with respect to probe (600), probe lock (960) of the present example does not lock automatically. Instead, as shown in FIG. 15 by an arrow, probe lock (960) is manually actuated by an operator to initiate locking. In particular, probe lock (960) is initially in the unlocked configuration. In this configuration, lock member (962) is oriented laterally to one side of chassis (906) such that unlock indicator (964) protrudes from the side of chassis (906). With lock member (962) in this position, unlock detent (974) is engaged with detent feature (903) to releasably hold lock member (962) in the locked position.

Once probe (900) is fully distally advanced such that prong (208) of holster (200) is fully engaged with cam feature (952), lock member (962) can be manually actuated to transition probe lock (960) to the locked configuration. This transition is initiated by an operator pressing unlock indicator (964) inwardly to move lock member (962) perpendicularly relative to the longitudinal axis of probe (900). This movement positions lock member (962) such that unlock indicator (964) is positioned generally within the perimeter of chassis (906), while lock indicator (966) is positioned generally outside the perimeter of chassis (906). In this position, each prong gap (965, 967) is also positioned away from prongs (208). Consequently, each indicator (964, 966) is positioned adjacent to the distal surface of each prong (208). Thus, each indicator (964, 966) physically locks each prong (208) into engagement with cam feature (952) to prevent decoupling of probe (900) from holster (200).

As described above, the transition from the unlocked configuration to the locked configuration can be tracked using tracking arm (970), cup (972), a magnet disposed within cup (972) and a hall effect sensor disposed within holster (200). In some operations, vacuum control module (400) can interpret signals from the hall effect sensor and generate a graphical indication of the status of probe lock (960) (e.g., locked or unlocked). By way of example only, such a graphical indication could be a graphical representation of a padlock in an unlocked or locked configuration. This graphical representation can also be color coded in some examples. In addition, it should be understood that in some examples, software in vacuum control module (400) can be coded with certain software lockout features to prevent use of holster (200) until probe (900) is locked thereto. Alternatively, vacuum control module (400) can be configured to sound an audible alarm if an operator attempts operation of holster (200) without probe (900) locked thereto. Regardless, it should be understood that tracking arm (970) and cup (972) can be used to provide yet another means of feedback to an operator to confirm that probe (900) is securely locked to holster (200).

Once probe (900) is coupled to holster (200) as described above, a biopsy procedure can be performed. At the conclusion of such a procedure, an operator may desire to decouple probe (900) from holster (200) for disposal of probe (900) and reuse of holster (200). To decouple probe (900) from holster (200), the procedure described above can be repeated. In particular, an operator can press lock indicator (966) to move lock member (962) back to the unlocked position shown in FIG. 15.

Once lock member (962) is moved, unlock indicator (964) indicates to an operator that probe (900) is unlocked by generally protruding beyond the outer perimeter of chassis (906). An operator can then remove probe (900) by pulling probe (900) proximally relative to holster (200) to disengage each prong (208) from each corresponding cam feature (952).

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A biopsy device comprising:
(a) a probe (600) including a probe body (606, 602), and a needle (610) extending distally from the probe body (606, 602);
(b) a holster (200) including a prong (208) extending from a portion of the holster (200), the probe (600) being releasably couplable to the holster (200); and
(c) a probe lock (660) including a lock member (662), the lock member (662) being configured to move laterally relative to a longitudinal axis defined by the needle (610) to both selectively lock the probe (600) to the holster (200) and to provide an affirmative indication of locking of the probe to the holster,
**characterized in that** the probe (600) further comprises a retainer (680) and the probe lock (660) further comprises a flex lock (670) extending from the lock member (662),
the flex lock (670) being configured to engage the retainer
(680) to hold the lock member (662) in an unlocked position and release the lock member (662) upon engagement with the prong (208).

2. The biopsy device of claim 1, the lock member (662) being configured to automatically transition from the unlocked position to a locked position via the prong (208) upon coupling of the probe (600) to the holster (200).

3. The biopsy device of claim 1, the flex lock (670) being resiliently biased to engage the retainer (680), the flex lock (670) being configured to disengage from the retainer (680) in response to engagement between the prong (208) of the holster (200) and the flex lock (670).

4. The biopsy device of claim 3, the lock member (662) being configured for manual actuation from a locked position to the unlocked position, the flex lock (670) being configured to engage the retainer (680) upon manual actuation of the lock member (662) to the unlocked position from the locked position.

5. The biopsy device of claim 1, the lock member (662) being configured to be manually actuated between the unlocked position and a locked position.

6. The biopsy device of any one or more of claims 1 through 5, the probe lock (660) being configured to provide affirmative feedback to an operator indicating when the probe is selectively locked to the holster via movement of the lock member (662).

7. The biopsy device of any one or more of claims 1 through 6, the lock member (662) being configured to extend outwardly from a portion of the probe body (606, 602) to provide affirmative feedback to an operator indicating when the probe (600) is selectively locked to the holster (200).

8. The biopsy device of any one or more of claims 1 through 7, the probe lock (660) being configured to transition between an unlocked configuration and a locked configuration, the lock member (662) being configured to extend from a first side of the probe body (606, 602) when the probe lock (660) is in the unlocked configuration, the lock member (662) being configured to extend from a second side of the probe body (606, 602) when the probe lock (660) is in the locked configuration.

9. The biopsy device of any one or more of claims 1 through 8, the probe lock (660) being configured to provide an audible sound to an operator indicating when the probe (600) is selectively locked to the holster (200).

10. The biopsy device of claim 1, the probe lock further including a resilient member (698), the flex lock (670) being configured to releasably fasten to the retainer (680) to hold the lock member (662) in the unlocked position against a resilient bias of the resilient member (698).

11. The biopsy device of claim 10, the retainer (680) including a ramp (684) and a flat surface (686), the flex lock (670) including a curved portion (672) terminating in a toothed end (674), the toothed end (674) of the flex lock (670) being configured to engage the flat surface (686) of the retainer (680) to hold the lock member (662) in the unlocked position against a resilient bias of the resilient member (698).

12. The biopsy device of claim 11, the ramp (684) of the retainer (680) being configured to flex the curved portion (672) of the flex lock (670) to thereby move the toothed end (674) into engagement with the flat surface (686) of the retainer (680).

13. The biopsy device of claims 11 or 12, the curved portion (672) of the flex lock (670) being configured to deform upon engagement of the toothed end (674) with at least a portion of the holster (200).

## Patentansprüche

1. Biopsievorrichtung, umfassend:
(a) eine Sonde (600), die einen Sondenkörper (606, 602) und eine Nadel (610), die sich distal vom Sondenkörper (606, 602) erstreckt, beinhaltet;
(b) ein Holster (200), das eine Klaue (208) beinhaltet, die sich von einem Abschnitt des Holsters (200) erstreckt, wobei die Sonde (600) lösbar mit dem Holster (200) koppelbar ist; und
(c) eine Sondenverriegelung (660), die ein Verriegelungselement (662) beinhaltet, wobei das Verriegelungselement (662) so konfiguriert ist, dass es sich seitlich relativ zu einer von der Nadel (610) definierten Längsachse bewegt, um sowohl die Sonde (600) selektiv am Holster (200) zu verriegeln als auch eine eindeutige Anzeige der Verriegelung der Sonde am Holster bereitzustellen,
**dadurch gekennzeichnet, dass** die Sonde (600) weiter eine Halterung (680) umfasst und die Sondenverriegelung (660) weiter eine flexible Verriegelung (670) umfasst, die sich vom Verriegelungselement (662) erstreckt,
wobei die flexible Verriegelung (670) so konfiguriert ist, dass sie mit der Halterung (680) in Eingriff kommt, um das Verriegelungselement (662) in einer entriegelten Position zu halten und das Verriegelungselement (662) bei Eingriff mit der Klaue (208) freizugeben.

2. Biopsievorrichtung nach Anspruch 1, wobei das Verriegelungselement (662) so konfiguriert ist, dass es beim Koppeln der Sonde (600) mit dem Holster (200) mittels der Klaue (208) automatisch von der entriegelten Position in eine verriegelte Position übergeht.

3. Biopsievorrichtung nach Anspruch 1, wobei die flexible Verriegelung (670) elastisch vorgespannt ist, um mit der Halterung (680) einzugreifen, wobei die flexible Verriegelung (670) so konfiguriert ist, dass sie sich als Reaktion auf den Eingriff zwischen der Klaue (208) des Holsters und der flexiblen Verriegelung (670) von der Halterung (680) löst.

4. Biopsievorrichtung nach Anspruch 3, wobei das Verriegelungselement (662) für eine manuelle Betätigung von einer verriegelten Position in die entriegelte Position konfiguriert ist, wobei die flexible Verriegelung (670) so konfiguriert ist, dass sie bei manueller Betätigung des Verriegelungselements (662) von der verriegelten Position in die entriegelte Position mit der Halterung (680) in Eingriff kommt.

5. Biopsievorrichtung nach Anspruch 1, wobei das Verriegelungselement (662) so konfiguriert ist, dass es manuell zwischen der entriegelten Position und einer verriegelten Position betätigt wird.

6. Biopsievorrichtung nach einem oder mehreren der Ansprüche 1 bis 5, wobei die Sondenverriegelung (660) so konfiguriert ist, dass sie einem Bediener durch Bewegung des Verriegelungselements (662) eine positive Rückmeldung bereitstellt, die anzeigt, wann die Sonde selektiv am Holster verriegelt ist.

7. Biopsievorrichtung nach einem oder mehreren der Ansprüche 1 bis 6, wobei das Verriegelungselement (662) so konfiguriert ist, dass es sich von einem Abschnitt des Sondenkörpers (606, 602) nach außen erstreckt, um einem Bediener eine positive Rückmeldung bereitzustellen, die anzeigt, wann die Sonde (600) selektiv am Holster (200) verriegelt ist.

8. Biopsievorrichtung nach einem oder mehreren der Ansprüche 1 bis 7, wobei die Sondenverriegelung (660) so konfiguriert ist, dass sie zwischen einer entriegelten Konfiguration und einer verriegelten Konfiguration übergeht, wobei das Verriegelungselement (662) so konfiguriert ist, dass es sich von einer ersten Seite des Sondenkörpers (606, 602) erstreckt, wenn sich die Sondenverriegelung (660) in der entriegelten Konfiguration befindet, wobei das Verriegelungselement (662) so konfiguriert ist, dass es sich von einer zweiten Seite des Sondenkörpers (606, 602) erstreckt, wenn sich die Sondenverriegelung (660) in der verriegelten Konfiguration befindet.

9. Biopsievorrichtung nach einem oder mehreren der Ansprüche 1 bis 8, wobei die Sondenverriegelung (660) so konfiguriert ist, dass sie einem Bediener einen hörbaren Ton bereitstellt, der anzeigt, wann die Sonde (600) selektiv am Holster (200) verriegelt ist.

10. Biopsievorrichtung nach Anspruch 1, wobei die Sondenverriegelung weiter ein elastisches Element (698) beinhaltet, wobei die flexible Verriegelung (670) so konfiguriert ist, dass sie lösbar an der Halterung (680) befestigt wird, um das Verriegelungselement (662) gegen eine elastische Vorspannung des elastischen Elements (698) in der entriegelten Position zu halten.

11. Biopsievorrichtung nach Anspruch 10, wobei die Halterung (680) eine Rampe (684) und eine flache Oberfläche (686) beinhaltet, wobei die flexible Verriegelung (670) einen gekrümmten Abschnitt (672) beinhaltet, der in einem gezahnten Ende (674) endet, wobei das gezahnte Ende (674) der flexiblen Verriegelung (670) so konfiguriert ist, dass es mit der flachen Oberfläche (686) der Halterung (680) in Eingriff kommt, um das Verriegelungselement (662) gegen eine elastische Vorspannung des elastischen Elements (698) in der entriegelten Position zu halten.

12. Biopsievorrichtung nach Anspruch 11, wobei die Rampe (684) der Halterung (680) so konfiguriert ist, dass sie den gekrümmten Abschnitt (672) der flexiblen Verriegelung (670) biegt, um dadurch das gezahnte Ende (674) in Eingriff mit der flachen Oberfläche (686) der Halterung (680) zu bewegen.

13. Biopsievorrichtung nach Anspruch 11 oder 12, wobei der gekrümmte Abschnitt (672) des flexiblen Verschlusses (670) so konfiguriert ist, dass er sich bei Eingriff des gezahnten Endes (674) mit mindestens einem Abschnitt des Holsters (200) verformt.

## Revendications

1. Dispositif de biopsie comprenant :
(a) une sonde (600) incluant un corps de sonde (606, 602), et une aiguille (610) s'étendant de manière distale à partir du corps de sonde (606, 602) ;
(b) un étui (200) incluant une broche (208) s'étendant depuis une partie de l'étui (200), la sonde (600) pouvant être couplée de manière amovible à l'étui (200) ; et
(c) un dispositif de verrouillage de sonde (660) incluant un élément de verrouillage (662), l'élément de verrouillage (662) étant configuré pour se déplacer latéralement par rapport à un axe longitudinal défini par l'aiguille (610) à la fois pour verrouiller sélectivement la sonde (600) sur l'étui (200) et pour fournir une indication positive de verrouillage de la sonde sur l'étui,
**caractérisé en ce que** la sonde (600) comprend en outre un dispositif de retenue (680) et le dispositif de verrouillage de sonde (660) comprend en outre un dispositif de verrouillage flexible (670) s'étendant à partir de l'élément de verrouillage (662),
le dispositif de verrouillage flexible (670) étant configuré pour venir en prise avec le dispositif de retenue (680) pour maintenir l'élément de verrouillage (662) dans une position déverrouillée et libérer l'élément de verrouillage(662) lors de la mise en prise avec la broche (208).

2. Dispositif de biopsie selon la revendication 1, l'élément de verrouillage (662) étant configuré pour passer automatiquement de la position déverrouillée à une position verrouillée par le biais de la broche (208) lors du couplage de la sonde (600) à l'étui (200).

3. Dispositif de biopsie selon la revendication 1, le dispositif de verrouillage flexible (670) étant sollicité de manière élastique pour venir en prise avec le dispositif de retenue (680), le dispositif de verrouillage flexible (670) étant configuré pour se désolidariser du dispositif de retenue (680) en réponse à la mise en prise entre la broche (208) de l'étui (200) et le dispositif de verrouillage flexible (670).

4. Dispositif de biopsie selon la revendication 3, l'élément de verrouillage (662) étant configuré pour un actionnement manuel d'une position verrouillée à la position déverrouillée, le dispositif de verrouillage flexible (670) étant configuré pour venir en prise avec le dispositif de retenue (680) lors de l'actionnement manuel de l'élément de verrouillage (662) vers la position déverrouillée depuis la position verrouillée.

5. Dispositif de biopsie selon la revendication 1, l'élément de verrouillage (662) étant configuré pour être actionné manuellement entre la position déverrouillée et une position verrouillée.

6. Dispositif de biopsie selon une ou plusieurs quelconques des revendications 1 à 5, le dispositif de verrouillage de sonde (660) étant configuré pour fournir un retour positif à un opérateur indiquant quand la sonde est sélectivement verrouillée sur l'étui par le biais du mouvement de l'élément de verrouillage (662).

7. Dispositif de biopsie selon une ou plusieurs quelconques des revendications 1 à 6, l'élément de verrouillage (662) étant configuré pour s'étendre vers l'extérieur depuis une partie du corps de sonde (606, 602) pour fournir un retour positif à un opérateur indiquant quand la sonde (600) est verrouillée sélectivement sur l'étui (200).

8. Dispositif de biopsie selon une ou plusieurs quelconques des revendications 1 à 7, le dispositif de verrouillage de sonde (660) étant configuré pour passer d'une configuration déverrouillée à une configuration verrouillée, l'élément de verrouillage (662) étant configuré pour s'étendre depuis un premier côté du corps de sonde (606, 602) lorsque le dispositif de verrouillage de sonde (660) est dans la configuration déverrouillée, l'élément de verrouillage (662) étant configuré pour s'étendre depuis un second côté du corps de sonde (606, 602) lorsque le dispositif de verrouillage de sonde (660) est dans la configuration verrouillée.

9. Dispositif de biopsie selon une ou plusieurs quelconques des revendications 1 à 8, le dispositif de verrouillage de sonde (660) étant configuré pour émettre un son audible à l'attention d'un opérateur indiquant quand la sonde (600) est sélectivement verrouillée sur l'étui (200).

10. Dispositif de biopsie selon la revendication 1, le dispositif de verrouillage de sonde incluant en outre un élément élastique (698), le dispositif de verrouillage flexible (670) étant configuré pour se fixer de manière amovible au dispositif de retenue (680) pour maintenir l'élément de verrouillage (662) dans la position déverrouillée contre une sollicitation élastique de l'élément élastique (698).

11. Dispositif de biopsie selon la revendication 10, le dispositif de retenue (680) incluant un plan incliné (684) et une surface plane (686), le dispositif de verrouillage flexible (670) incluant une partie incurvée (672) se terminant par une extrémité dentée (674), l'extrémité dentée (674) du dispositif de verrouillage flexible (670) étant configurée pour venir en prise avec la surface plane (686) du dispositif de retenue (680) pour maintenir l'élément de verrouillage (662) dans la position déverrouillée contre une sollicitation élastique de l'élément élastique (698).

12. Dispositif de biopsie selon la revendication 11, le plan incliné (684) du dispositif de retenue (680) étant configuré pour fléchir la partie incurvée (672) du dispositif de verrouillage flexible (670) pour ainsi déplacer l'extrémité dentée (674) en prise avec la surface plane (686) du dispositif de retenue (680).

13. Dispositif de biopsie selon les revendications 11 ou 12, la partie incurvée (672) du dispositif de verrouillage flexible (670) étant configurée pour se déformer lors de la mise en prise de l'extrémité dentée (674) avec au moins une partie de l'étui (200).
